# EUROPEAN PATENT APPLICATION

(11) **EP 4 789 674 A1**
(43) Date of publication of application: **12.08.2026**
(21) Application number: 24874754.5
(22) Date of filing: 04.10.2024
(51) Int. Cl.: A61K 35/28, A61P 43/00, C12N 5/10, C12N 5/0775

(54) **COMPOSITION FOR TREATING AND/OR PREVENTING MITOCHONDRIAL DISEASE INCLUDING HIGH-PURITY MESENCHYMAL STEM CELLS**

(30) Priority: 05.10.2023 JP 2023173545
(71) Applicant: PUREC CO., LTD., Izumo-shi, Shimane 693-0021 (JP); National University Corporation Shimane University, Matsue-shi, Shimane 690-8504 (JP)
(72) Inventor: TAKETANI Takeshi, Izumo-shi, Shimane 693-0021 (JP); YANG Jiahao, Izumo-shi, Shimane 693-0021 (JP); IYOKU MATSUZAKI Yumi, Izumo-shi, Shimane 693-0021 (JP); SUYAMA Takashi, Izumo-shi, Shimane 693-0021 (JP)
(74) Representative: J A Kemp LLP
(86) International application number: PCT/JP2024/035635
(87) International publication number: WO 2025/075154

(57) **Abstract**

In an embodiment, the present invention relates to a composition for treating and/or preventing mitochondrial disease. In an embodiment, the present invention relates to an agent for improving mitochondrial dysfunction.

## Description

### Technical Field

The present invention relates to a composition for treating and/or preventing mitochondrial disease comprising high-purity mesenchymal stem cells.

### Background Art

Pathological conditions in which mitochondrial dysfunction develops clinical symptoms are generically referred to as mitochondrial disease. Mitochondria, which are present in cells throughout the body, have the function to generate energy. Deterioration in mitochondrial function therefore leads to pathological conditions such as energy metabolism disorder in various organs.

Examples of causes of mitochondrial disease include mutations of genes of nuclear DNAs and abnormalities of mitochondrial DNAs (mtDNA). Around 200 genetic mutations have been identified in genes on nuclear DNAs. More than 100 pathological point mutations have been identified on mtDNAs.

Regarding mitochondrial disease, for example, Patent Literature 1 describes a pharmaceutical composition for treating or preventing mitochondrial disease comprising a specific compound. Patent Literature 2 describes a pharmaceutical composition for mitochondrial disease and the like, comprising human stem cells enriched with functional mitochondria. Unfortunately, it is only taurine that is a therapeutic drug strictly certified as effective in clinical trials in Japan at present. The approved purposes of taurine are limited to "suppression of a stroke-like episode in mitochondrial myopathy, encephalopathy, lactic acidosis, and stroke-like episodes (MELAS)". Any drug effective against mitochondrial disease is not present currently.

### Citation List

### Patent Literature

Patent Literature 1:
   Japanese Patent Application No. 2022-125211
Patent Literature 2:
   Japanese Patent Application No. 2021-202844

### Non Patent Literature

Non Patent Literature 1
Elad Jacoby et al., npj Regenerative Medicine volume 6, Article number: 58 (2021)

### Summary of Invention

In an embodiment, an object of the present invention is to provide a composition for treating and/or preventing mitochondrial disease. In an embodiment, an object of the present invention is to provide an agent for improving mitochondrial dysfunction.

The present inventor has found that a cell population of an LNGFR (CD271)-positive or LNGFR (CD271)/Thy-1 (CD90) double-positive rapidly proliferating mesenchymal stem cell clone or progeny thereof may be effective in treating and/or preventing mitochondrial disease.

The present invention includes the following embodiments.
(1) A composition for treating and/or preventing mitochondrial disease, comprising a cell population of an LNGFR (CD271)-positive or LNGFR (CD271)/Thy-1 (CD90) double-positive rapidly proliferating mesenchymal stem cell clone or progeny thereof.
(2) The composition according to (1), wherein the mitochondrial disease is MELAS (mitochondrial myopathy, encephalopathy, lactic acidosis, and stroke-like episodes).
(3) The composition according to (1) or (2), wherein the coefficient of variation of forward scatter from the cell population or the progeny thereof by flow cytometry is 40% or less.
(4) The composition according to any of (1) to (3), wherein the cell population is derived from human bone marrow.
(5) The composition according to any of (1) to (4), wherein the cell population is obtained by a method, comprising:
   (a) obtaining a population of LNGFR (CD271)-positive or LNGFR (CD271)/Thy-1 (CD90) double-positive mesenchymal stem cells;
   (b) subjecting clones of the mesenchymal stem cells to single-cell culture; and
   (c) collecting a clone that proliferates rapidly and subjecting the clone to expansion culture.
(6) The composition according to (5), wherein the method further comprises: (d) measuring the coefficient of variation of forward scatter by flow cytometry, and (e) collecting a population wherein the coefficient of variation of forward scatter by flow cytometry is 40% or less.
(7) The composition according to any of (1) to (6), wherein the composition is intravenously administered to a subject.
(8) An agent for improving mitochondrial dysfunction, comprising a cell population of an LNGFR (CD271)-positive or LNGFR (CD271)/Thy-1 (CD90) double-positive rapidly proliferating mesenchymal stem cell clone or progeny thereof.
(9) A method for treating and/or preventing mitochondrial disease, comprising administering a cell population of an LNGFR (CD271)-positive or LNGFR (CD271)/Thy-1 (CD90) double-positive rapidly proliferating mesenchymal stem cell clone or progeny thereof to a subject.
(10) The method according to (9), wherein the mitochondrial disease is MELAS (mitochondrial myopathy, encephalopathy, lactic acidosis, and stroke-like episodes).
(11) The method according to (9) or (10), wherein the coefficient of variation of forward scatter from the cell population or the progeny thereof by flow cytometry is 40% or less.
(12) The method according to any of (9) to (11), wherein the cell population is derived from human bone marrow.
(13) The method according to any of (9) to (12), wherein the cell population is obtained by a process comprising:
   (a) obtaining a population of LNGFR (CD271)-positive or LNGFR (CD271)/Thy-1 (CD90) double-positive mesenchymal stem cells;
   (b) subjecting clones of the mesenchymal stem cells to single-cell culture; and
   (c) collecting a clone that proliferates rapidly and subjecting the clone to expansion culture.
(14) The method according to (13), wherein the process for obtaining the cell population further comprises: (d) measuring the coefficient of variation of forward scatter by flow cytometry, and (e) collecting a population wherein the coefficient of variation of forward scatter by flow cytometry is 40% or less.
(15) A method for improving mitochondrial dysfunction, comprising administering a cell population of an LNGFR (CD271)-positive or LNGFR (CD271)/Thy-1 (CD90) double-positive rapidly proliferating mesenchymal stem cell clone or progeny thereof to a subject.

The present invention can provide a composition for treating and/or preventing mitochondrial disease. The present invention can also provide an agent for improving mitochondrial dysfunction.

### Brief Description of Drawings

[Figure 1] Figure 1 shows a schematic diagram of a process involving establishing iPS cells from a mitochondrial disease patient and differentiating the iPS cells into nerve cells.
[Figure 2] Figure 2 shows the results of measuring the gene expression of OCT-4, TRA1-50, and NANOG in iPS cells derived from a MELAS patient.
[Figure 3] Figure 3 shows the results of measuring the gene expression and the gene mutation of NeuO in nerve cells derived from a MELAS patient.
[Figure 4] Figure 4 shows the capacities of RECs and MECs to transfer mitochondria to MELAS-N cells.
[Figure 5] Figure 5 shows the mitochondrial reactive oxygen-reducing effect of RECs and MECs in MELAS-N cells.
[Figure 6] Figure 6 shows the respiratory function recovery effect of RECs and MECs in MELAS-N cells.
[Figure 7] Figure 7 shows the GDF-15-reducing effect of RECs and MECs in MELAS-N cells.
[Figure 8] Figure 8 shows the contents and the properties of mitochondria in RECs and MSCs. Figure 8A shows the results of quantification of mitochondria. Figure 8B shows the results of quantification of swollen mitochondria. Figure 8C shows the results of the cellular respiratory activity.
[Figure 9] Figure 9 shows the results of measuring the amount of REC-derived mitochondria transferred to mouse tissues one week after the RECs are intravenously administered to mitochondrial disease model mice.

### Description of Embodiments

In an embodiment, the present invention relates to a composition for treating and/or preventing mitochondrial disease, comprising a cell population of an LNGFR (CD271)-positive or LNGFR (CD271)/Thy-1 (CD90) double-positive rapidly proliferating mesenchymal stem cell clone or progeny thereof.

### Process for obtaining mesenchymal stem cells

In the present invention, a process for obtaining LNGFR (CD271)-positive or LNGFR (CD271)/Thy-1 (CD90) double-positive mesenchymal stem cells can be performed, for example, in accordance with a process described in WO2009/31678 or WO2021/144995. An overview of the process for obtaining the mesenchymal stem cells is as follows.

An LNGFR (CD271)-positive (CD271+) or CD271/CD90 (Thy-1) double-positive (CD271+CD90+) cell fraction is sorted from a cell population comprising human mesenchymal stem cells, resulting in highly concentrating the mesenchymal stem cells. If a cell population comprising human mesenchymal stem cells comprises hematopoietic cells, a step of sorting out CD45/CD235a double-negative (CD45-CD235a-) cells may be added to sort out non-hematopoietic cells.

Examples of materials for obtaining a cell population comprising human mesenchymal stem cells include, but not limited to, bone marrow and peripheral blood (including peripheral blood after the administration of G-CSF). Examples of usable bone marrow include bone marrow from the vertebra, the sternum, and the ilium. For example, mononuclear cells prepared from human bone marrow or fat/placenta chorion are usable. A cell population may be selected beforehand from these materials by flow cytometry or affinity chromatography using known markers for mesenchymal stem cells (for example, CD105(SH2), CD73(SH3/4), CD71, and CD44).

In an embodiment, the human mesenchymal stem cells are obtained or derived from a patient to be treated for mitochondrial disease and/or to be prevented from developing mitochondrial disease. In another embodiment, the human mesenchymal stem cells are obtained or derived from a donor different from a patient to be treated for mitochondrial disease and/or to be prevented from developing mitochondrial disease.

If the material is cell clusters comprising mesenchymal stem cells in the preparation of the cells, the material can be subjected to physical treatment such as pipetting or enzymatic treatment such as trypsin or collagenase as needed. If red blood cells are mixed in the material, it is preferable that the red blood cells be hemolyzed beforehand. CD271+ cells or CD271+CD90+ cells are sorted out from the prepared cell population as mentioned above.

Examples of the method for sorting out CD271+ cells or CD271+CD90+ cells include a method using an antibody. The antibody is an anti-CD271 antibody and/or an anti-CD90 antibody that enables sorting out CD271+ cells or CD271+CD90+ cells. If flow cytometry is used for the sorting, anti-CD271 antibodies, or an anti-CD271 antibody and an anti-CD90 antibody labeled with different fluorescent dyes such as FITC, PE, and APC can be suitably used in combination to sort viable cells within a short time. CD271+CD90+ cells can be sorted by a method using magnetic beads or a method using affinity chromatography as well as by flow cytometry.

Fluorescent dye that stains dead cells (for example, PI) may be reacted beforehand with the cell population before the use of these methods, followed by removal of cells stained fluorescently, resulting in removal of the dead cells.

### Concentration of REC cells

The sorted LNGFR-positive cells or LNGFR/Thy1 double-positive cells are then subjected to single-cell culture (cloning), followed by the selection of a rapidly proliferating lot, resulting in obtaining high-purity human mesenchymal stem cells (REC: rapidly expanding clone), which are superior in proliferative capacity, differentiation potential, and migration capacity.

LNGFR-positive cells or LNGFR-positive and Thy1-positive cells are first clone-sorted in a 96-well plate using flow cytometry (a cell sorter). That is, one cell is seeded in each well. Two weeks after the single-cell culture, the culture plate is photographed under a microscope, followed by selecting confluent or semiconfluent wells. The cells comprised in the wells are used as RECs.

The terms "proliferate rapidly" and "rapidly proliferating" mean that if one cell is seeded in each well of a 96-well plate for culture, the cell has such a growth rate (for example, a doubling time of 26 ± 1 hours) that the culture plate is confluent or semiconfluent two weeks after the beginning of the culture or earlier.

For example, the term "confluent" means that 90% or more of the surface of a culture vessel (culture surface) is covered with the cultured cells. The term "semiconfluent" means that 70 to 90% of the surface of a culture vessel (culture surface) is covered with the cultured cells. The sizes and the types of the culture implements used can be suitably changed depending on the growth rate of the cells. Slowly proliferating cells (moderately/slowly expanding cells), that is, cells that were not semiconfluent or confluent two weeks after the single-cell culture, are discarded. The RECs selected as the REC and collected from each well are poured into a culture flask and further cultured until the confluence (expansion culture). The cells subjected to the expansion culture are then collected separately. The RECs derived from one well are considered as one lot. These may be used in the below-described selection.

Since the cells of the present invention were obtained by the clone sorting, involving seeding one cell in each well, the proliferated cells are the same in all heritages. In the present invention, the entire cell population may therefore be referred to as a "clone", and an individual cell constituting the cell population may be referred to as a "clone".

In the present invention, the RECs to be used in the selection can be evaluated beforehand with an REC marker (anti-Ror2). For example, after the expansion culture, the adherently proliferated cells are collected from all the lots. Some cells (around 1 to 3 × 10⁵ cells) of each lot are separated to be single-stained with a monoclonal antibody to anti-Ror2. The technique involving single-staining with a monoclonal antibody to anti-Ror2 (WO2016/17795) is known. In brief, the rate of the REC marker-positive cells in the collected cells is determined by flow cytometry analysis using the REC marker. The rate may be determined by quantifying the expression of mRNA of Ror2 using quantitative PCR. The rate may also be manually determined through a microscope. A lot (cell population) having the above-mentioned positive rate of a certain value (for example, 65%) or more is recognized as acceptable. This can also be used in the selection described later.

The progeny of the cell population described herein can also be used in the present invention. The progeny can be prepared by the subculture of the cell population. Although the progeny may be LNGFR (CD271) - positive (CD271+) or CD271/CD90 (Thy-1) double-positive (CD271+CD90+), the progeny may be lacking in expression of these markers in the process of the culture. The cells lacking in expression of the markers may be selected based on the differentiation potential, the proliferative capacity, the cell size, and/or the uniformity thereof.

### Selection of stem cell population of the present invention

The cell population described herein or the cell population thereof may be further selected using the coefficient of variation (CV value) of the forward scatter in flow cytometry and/or the average size of the cells as an index.

The forward scatter refers to light forward scattered at a small angle to the axis of laser light. The forward scatter, which comprises scattered laser light, diffracted laser light, and refracted laser light generated on the surfaces of cells, provides information on the size of a sample.

The coefficient of variation (CV), which refers to a value obtained by dividing the standard deviation by the average, is a value for relatively evaluating the relationship between the variations of data expressed in different units or the relationship between data and the variation to the average.

In the present invention, a cell population wherein the above-mentioned CV value is 40% or less may be selected. The cell population wherein the CV value is 40% or less comprises cells having a uniform size. The CV value is preferably 35% or less, 30% or less, 25% or less, or 20% or less.

The cell population selected by the present invention may have an average cell size of 20 µm or less. The cell size is, for example, 18 µm or less, and is in the range of 14 to 18 µm.

The number of the clones constituting the cell population selected as described above may be any number. For example, 1 mL of the solution may comprise around 0.8 × 10⁷ to 1.2 × 10⁷ cells.

Since a cell population of FSCs having a low CV value may be superior in proliferative capacity and differentiation potential, the use of such a cell population may enable exerting a higher effect of improving mitochondrial disease.

### Purposes

The cell population described herein or the progeny thereof can be used for treating and/or preventing mitochondrial disease as a therapeutic mesenchymal stem cell population. For example, the cell population described herein or the progeny thereof can be administered to a subject to exert at least one, for example, two, three, or four, of, for example, the effects of increasing the amount of mitochondria, suppressing the swelling of mitochondria, reducing reactive oxygen species in mitochondria, improving the mitochondrial function of producing ATP, and improving the mitochondrial respiratory function in the administration subject as compared with a control (to which the cell population or the progeny is not administered).

In an embodiment, the present invention relates to a pharmaceutical composition comprising a cell population described herein or progeny thereof. The cell population or the progeny thereof may be comprised in the pharmaceutical composition described herein at any concentration. If the pharmaceutical composition is liquid, the concentration may be 1 × 10³ cells/ml or more, 1 × 10⁴ cells/ml or more, or 1 × 10⁷ cells/ml or more, and may be 1 × 10⁹ cells/ml or less, 1 × 10⁸ cells/ml or less, or 1 × 10⁷ cells/ml or less. For example, the concentration may be 1 × 10³ to 1 × 10⁹ cells/ml, 1 × 10⁴ to 1 × 10⁸ cells/ml, 1 × 10⁵ to 1 × 10⁷ cells/ml, or about 1 × 10⁶ cells/ml. The pharmaceutical composition described herein may comprise one or more pharmaceutically acceptable carriers besides the cell population or the progeny thereof. Examples of the carriers include extenders, diluents, buffers, isotonizing agents, chelating agents, thickeners, coloring agents, stabilizers, emulsifiers, dispersants, suspending agents, and antiseptics. The pharmaceutical composition described herein may be in any dosage form. For example, the pharmaceutical composition can be prepared into an injection such as sterile solution or a suspension in accordance with a common method.

In an embodiment, the present invention relates to a pharmaceutical composition for treating and/or preventing mitochondrial disease comprising a cell population described herein or progeny thereof. In another embodiment, the present invention relates to a method for treating and/or preventing mitochondrial disease comprising administering a cell population described herein or progeny thereof to a subject.

The "subject" to which the pharmaceutical composition, the cell population or the progeny thereof described herein is administered means a human or a non-human organism. Examples of the non-human organism include birds and non-human mammals (for example, bovines, simians, cats, mice, rats, guinea pigs, hamsters, pigs, dogs, rabbits, sheep, and horses).

The "treating" as used herein includes all the mitochondrial disease-related treatments such as the maintenance of improved symptoms, the suppression of recurrence, and a reduction in other treatments besides improvement in mitochondrial disease-related symptoms. The "preventing" as used herein includes preventing the development of mitochondrial disease-related symptoms or reducing the risk thereof.

The mitochondrial disease as used herein means pathological conditions in which clinical symptoms develop due to the impairment of mitochondrial function. Examples of mitochondrial disease include MELAS (mitochondrial myopathy, encephalopathy, lactic acidosis, and stroke-like episodes), Kearns-Sayre syndrome (KSS), CPEO (chronic progressive external ophthalmoplegia), MERRF (myoclonus epilepsy associated with ragged-red fibers), sensorineural hearing loss, fatal infantile encephalopathy, Leigh/Leigh-like syndrome, Leber disease (Leber hereditary optic neuropathy [LHON]), Barth syndrome, Pearson's disease, and Friedreich ataxia (FRDA). For example, mitochondrial disease may be MELAS.

In an embodiment, the present invention relates to an agent for improving mitochondrial dysfunction comprising the cell population described herein or the progeny thereof. In another embodiment, the present invention relates to a pharmaceutical composition for improving mitochondrial dysfunction comprising the cell population described herein or the progeny thereof. In yet another embodiment, the present invention relates to a method for improving mitochondrial dysfunction comprising administering the cell population described herein or the progeny thereof to a subject.

The mitochondrial dysfunction as used herein means at least one, for example, two, three, or four, of a decrease in the amount of mitochondria, the swelling of mitochondria, an increase in reactive oxygen species in mitochondria, a deterioration in mitochondrial ATP production function, and mitochondrial respiratory hypofunction.

The dose, the frequency of administrations, the number of administrations, and the route of administration of the cell population or the progeny thereof, the pharmaceutical composition, or an agent for improving mitochondrial dysfunction described herein can be selected depending on the age, the sex, the body weight, the symptoms, the route of administration, the frequency of administration of a subject, and the dosage form. An effective dose is an amount required for treating and/or preventing mitochondrial disease, or improving mitochondrial dysfunction. The dose of the cell population or the progeny thereof, the pharmaceutical composition, or an agent for improving mitochondrial dysfunction may be any amount. For example, the amount of the cell population may be 10⁴ cells/kg or more, 10⁵ cells/kg or more, or 10⁶ cells/kg or more, and may be 10¹⁰ cells/kg or less, 10⁹ cells/kg or less, or 10⁸ cells/kg or less. For example, the amount may be 10⁴ cells/kg to 10¹⁰ cells/kg, 10⁵ cells/kg to 10⁹ cells/kg, 10⁶ cells/kg to 10⁸ cells/kg, or 10⁷ cells/kg. For example, the frequency of administrations may be once or more per day, once or more per two days, or once or more per three days, and may be once or less per two months, once or less per month, once or less per two weeks. For example, the frequency may be once per week. The number of administrations may be once or more, twice or more, or three times or more, and may be seven times or less, six times or less, or five times or less. For example, the number may be four times. The route of administration may be intravenous administration, intramuscular administration, intraperitoneal administration, or subcutaneous administration.

In an embodiment, the present invention relates to a process for producing a composition for treating and/or preventing mitochondrial disease, comprising obtaining a cell population described herein or progeny thereof, and preparing a pharmaceutical composition described herein comprising the cell population or the progeny thereof. In another embodiment, the present invention relates to a method for treating and/or preventing mitochondrial disease comprising administering a cell population described herein or progeny thereof to a subject.

### Examples

### <Example 1>

### Cells

Nerve cells (MELAS-N and Control-N) were differentiated from iPS cells established from three MELAS patients and a normal healthy person for use. The iPS cells (201B7, HSP-0063) established from a normal healthy person and the iPS cells (HSP-2864, HSP-0458, and HSP-0461) established from the MELAS patients were obtained from the RIKEN CELL BANK (Japan).

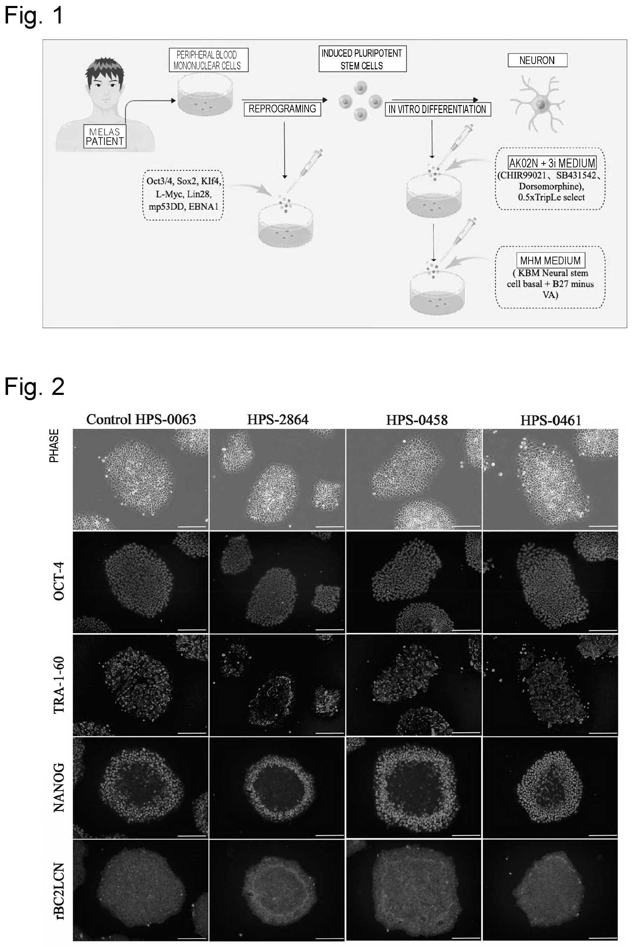

Figure 1 shows a schematic diagram of a process involving establishing iPS cells from a mitochondrial disease patient and differentiating the iPS cells into nerve cells. Specifically, OCT3/4, SOX2, KLF4, L-MYC, LIN28, mp53DD, and EBNA1 were introduced into peripheral blood monocytes with episomal vectors to establish the iPS cells (K. Fujimori et al., Stem Cell Reports, 9(2017) 1675-1691). The neurodifferentiation from the iPS cells was performed in accordance with the method of K. Fujimori et al (mentioned above). Specifically, the iPS cells were cultured in StemFit AK02N medium comprising three inhibitors (3 µM CHIR-99021 (REPROCELL Inc., Japan), 3 µM SB-431542 (FUJIFILM Corporation, Japan), and 3 µM dorsomorphin (Sigma, USA). The cultured cells were dissociated with TrypLE Select five days after, followed by culture in MHM medium (KBM Neural Stem Cell Kit, Kohjin Bio Co., Ltd., Japan) comprising 2% B27-Minus vitamin A (Thermo Fisher Scientific Inc., USA) and 20 ng/ml bFGF (Thermo Fisher Scientific K.K., Japan). The method and other details were performed in accordance with K. Fujimori et al (mentioned above).

Frozen RECs worthy of GMP were purchased from PuREC Co., Ltd. LNGFR/Thy-1 double-positive cells selected from bone marrow mononuclear cells were seeded in a 96-well plate as single cells, followed by obtaining the RECs as rapidly proliferating cells that reached confluence after two weeks. MSCs were purchased from Lonza K.K.

### Culture of nerve cells

The nerve cells (MELAS-N and Control-N) differentiated from the iPS cells established from the three MELAS patients and the normal healthy person were cultured in **MEF** feeder cell-free StemFit AK02N medium (Ajinomoto Co., Inc., Japan) while remaining undifferentiated. The iPS cells were dissociated with TrypLE select (Thermo Fisher Scientific Inc., USA) into single cells, which were reseeded in a 6-well plate treated with iMatrix511 (Nippi, Incorporated, Japan) at a density of 2 × 10⁴ cells per plate and cultured in StemFit AK02N comprising Y27632 (FUJIFILM Corporation, Japan) under the conditions of 5% CO₂ and 37°C. In order to remove Y27632, the medium was replaced on the day following the subculture, followed by medium replacement every two to three days.

Gene expression in iPS cells derived from MELAS patients and properties of nerve cells derived from MELAS patients

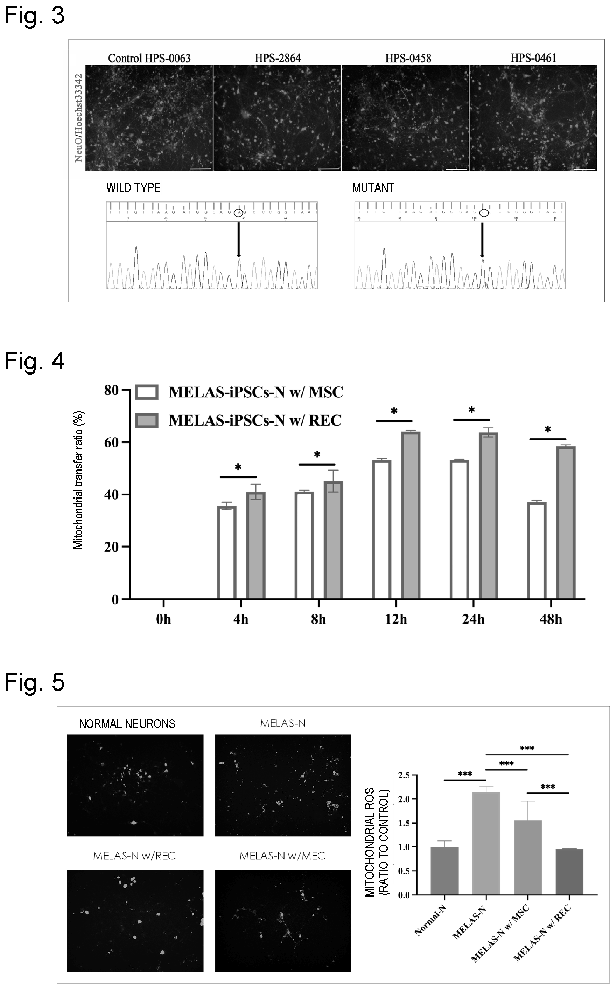

The gene expression of OCT-4, TRA1-50, and NANOG as stem cell markers were examined in the iPS cells derived from the MELAS patients. Figure 2 shows the results. The gene expression and the gene mutation of NeuO as a nerve cell-specific marker were examined in the nerve cells derived from the MELAS patients. Figure 3 shows the results.

### Transfer of mitochondria to MELAS-N cells

The MELAS-N cells were labeled with 5 µL/mL of Vybrant^{™} DID Cell-Labeling Solution (Thermo Fisher Scientific Inc., USA) at 37°C for 20 minutes. The MSCs and RECs were labeled with 200 nM MitoTracker^{™} Green FM (Thermo Fisher Scientific Inc., USA) at 37°C for 20 minutes. The labeled MELAS-N cells were cocultured with the labeled MSCs or RECs in RPMI1640 growth medium (FUJIFILM Wako Pure Chemical Corporation, Japan) comprising 1% penicillin-streptomycin and 10% fetal bovine serum (FBS; HyClone) for 48 hours. The cells were then washed with phosphate buffered saline (PBS) and incubated in TrypLE^{™} Express Enzyme (Thermo Fisher Scientific Inc., USA) at 37°C for 5 minutes to be detached. The cell viability was evaluated using the 4% trypan blue exclusion method and a light microscope, and was always over 95%. The cells were obtained with CytoFLEX (Beckman Coulter, Inc., USA). The data was analyzed by FlowJO^{™} software version 10 (Becton, Dickinson and Company, USA). Mitochondria-specific migration of the dye from the MSCs or the RECs was measured by gating DiD events, excluding doublets (forward scatter width vs. height), and measuring fluorescence through a fluorescein channel.

Figure 4 shows the results. As shown in Figure 4, the RECs were advantageously higher in the capacity to transfer mitochondria to the MELAS-N cells than MSCs.

### Reduction of reactive oxygen

The production of mitochondrial superoxide was visualized with the red fluorescent probe MitoSOX^{™} (Thermo Fisher Scientific Inc., USA) in accordance with the manufacture's protocol. Specifically, the cells (1 × 10⁵) proliferated on a 24-well plate were washed with PBS twice, followed by removal of the medium. The cells were incubated with 5 µM MitoSOX^{™} working solution at 37°C for 10 minutes. The cells were then washed with warm buffer (Thermo Fisher Scientific Inc., USA) gently three times, followed by imaging the cells through a fluorescent microscope (BZ-X710, KEYENCE CORPORATION, Japan) immediately. In order to confirm that MitoSOX^{™} was localized in mitochondria, the cells were labeled with 0.1 µmol/L MitoBright LT-Green (DOJINDO LABORATORIES, Japan) at 37°C for 15 minutes. The average fluorescence intensities of MitoSOX-Red and MitoBright LT-Green were detected with a GloMax(R) Discover Microplate Reader (Promega Corporation, USA).

Figure 5 shows the results. As shown in Figure 5, although the level of the reactive oxygen species was high in the MELAS-N, the level of the reactive oxygen species was reduced by the RECs and the MSCs, and the RECs were advantageously higher in the degree of reduction thereof than the MSCs.

### Respiratory function recovery

The oxygen consumption rate (OCR) was directly measured with a Seahorse XF HS mini (Agilent technologies, USA) to measure important parameters of mitochondrial functions. Specifically, the cells in each group were seeded in an XF cell culture microplate (Agilent technologies, USA) at a density of 15,000 cells per well in accordance with the manufacture's protocol. After overnight culture, the medium was replaced with Seahorse XF RPMI medium (Agilent technologies, USA) comprising 10 mM glucose, 2 mM L-glutamine, and 1 mM sodium pyruvate (pH 7.4). The cells were transferred to a non-CO₂ incubator and left to stand in the incubator for 60 minutes. In a Mito Stress assay, the cells were treated by sequentially adding 1.5 µM/well oligomycin (Olig), 1 µM/well FCCP, and 0.5 µM/well rotenone/antimycin A (AA/Rot). The plotted values were expressed as the percentage of the basal oxygen consumption rate in the real-time evaluation of mitochondrial respiration. The results were analyzed by XFe Wave software (Agilent technologies, USA).

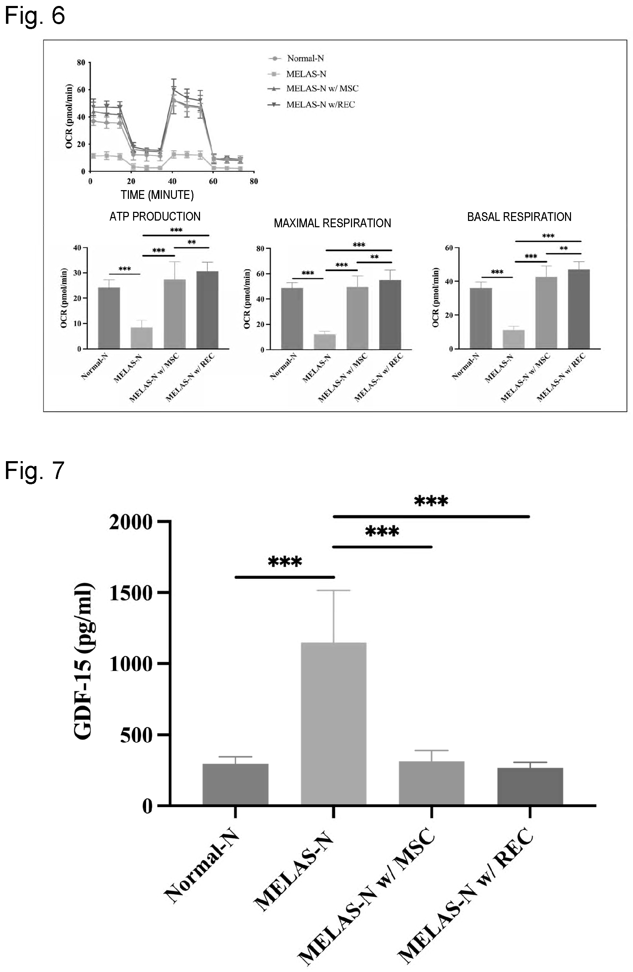

Figure 6 shows the results. As shown in Figure 6, although the oxygen consumption rate (OCR) of MELAS-N was low, the OCR level was increased by the OCRs and the MSCs. The RECs were advantageously higher in the degree of an increase therein than the MSCs.

### Decrease in GDF-15

The cell culture medium supernatant was prepared in accordance with the manufacture's protocol. The cell culture medium supernatant was prepared with an enzyme-linked immunosorbent assay (ELISA) kit (Thermo Fisher Scientific Inc., USA) in accordance with the manufacture's protocol, followed by measurement of GDF-15 as a biomarker for mitochondrial disease. The optical densities of the samples were measured at 450 nm with a GloMax(R) Discover Microplate Reader (Promega K.K., USA).

Figure 7 shows the results. As shown in Figure 7, although the expression level of GDF-15 was high in MELAS-N, the coculture with the RECs and the MSCs reduced the expression level of GDF-15.

### Comparison between RECs and MSCs

### Quantification of mitochondria

Three REC clones and three MSC clones were labeled with 200 nM MitoTracker^{™} Green FM (Thermo Fisher Scientific Inc., USA) at 37°C for 20 minutes. The average fluorescence intensity of MitoTracker^{™} Green FM was detected by flow cytometry (CytoFLEX, Beckman Coulter, Inc., USA), followed by the quantification of the data using FlowJo^{™} software version 10 (Becton, Dickinson and Company, USA).

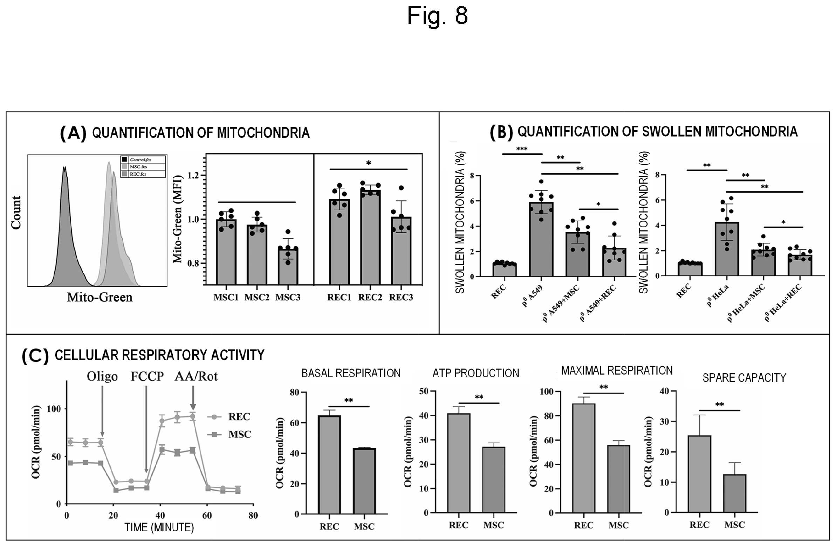

Figure 8 shows the results. As shown in Figure 8A, the amount of mitochondria was larger in the RECs than in the MSCs.

### Quantification of swollen mitochondria

Three REC clones and three MSC clones were proliferated on 35 mm dishes for 10 to 12 hours. Then, the cells were fixed beforehand with 2.5% glutaraldehyde solution for electron microscopy (FUJIFILM Wako Pure Chemical Corporation, Japan), 2% PFA, and 0.1 M phosphate buffer (FUJIFILM Wako Pure Chemical Corporation, Japan) at room temperature for two hours and washed with water three times. The cells were washed with PBS and dehydrated with ethanol liquids at gradually varying concentrations (50%, 70%, 90%, 95%, and 100%) for 8 minutes. The cells were then attached to epoxy resin using propylene oxide to facilitate the permeation, followed by curing at 60°C for 24 hours. Ultrathin sections were collected with a diamond knife, mounted on a copper mesh grid, stained with uranyl acetate, and imaged through the transmission electron microscope Topcon EB-002B (KEYENCE CORPORATION, Japan). The swelling of mitochondria was quantitatively analyzed from the obtained images by Image J software (Wyne Rasband, USA).

Figure 8 shows the results. As shown in Figure 8B, although many swollen cells were present in the MELAS-N, the swollen cells were reduced with the RECs and the MSCs. The RECs were advantageously higher in the degree of reduction thereof than the MSCs.

### Mitochondrial respiratory function

The oxygen consumption rate (OCR) was examined with a Seahorse XF Cell Mito Stress Test Kit (Agilent technologies, USA) to be measured with a flux analyzer (Seahorse XF HS mini, Agilent technologies, USA) in accordance with the protocol. Specifically, the cells were uniformly seeded in an XF HSmini cell culture plate (1.5 × 10⁴ cells/well). The cells were equilibrated with XF RPMI medium (Agilent technologies, USA) supplemented with 10 mM glucose, 1 mM sodium pyruvate, and 2 mM 1-glutamine, transferred to a non-CO₂ incubator, and left to stand for one hour. Subsequently, the coupled respiration and the uncoupled respiration were then measured with 1.5 µM oligomycin (Olig). The maximum respiratory capacity (maximal respiration) was determined with 1 µM FCCP. Finally, the non-mitochondrial respiration was measured with the combination of 0.5 µM antimycin A and rotenone (AA/Rot). The plotted values were expressed as the percentage of the basal OCR in the real-time evaluation of mitochondrial respiration. The data set was analyzed by Wave software (Agilent technologies, USA).

Figure 8 shows the results. As shown in Figure 8C, the coculture with the RECs and the MSCs restored the respiratory function of MELAS-N to the level of normal nerve cells. The RECs were significantly higher in the effect of restoring the respiratory function than MSCs.

### <Example 2>

### In vivo mitochondrial transfer test

It was examined and proved whether, in mitochondria-deficient model mice (ND6 Mitomice), which deteriorated in mitochondrial function due to ND6 gene mutation, REC-derived mitochondria transferred to organs, and restored the mitochondrial function *in* vivo. If this test proves the effectiveness, it is conceivable that the RECs can be an effective treatment for patients with mitochondrial disease, for whom any treatment is not established.

### Treatment of animals

Two groups of eight to nine-week-old ND6 Mitomice (four mice in each group) were provided, followed by tail vein injection under anesthesia (isoflurane) [pain category: B]. 2 × 10⁶ RECs were suspended in 0.2 mL of a solvent in which HSA-CP-1 and 0.5% HSA/RPMI were mixed in equal amounts, followed by administration of the suspension to a REC-treated group. 0.2 mL of the solvent only was administered to a control group. One to two weeks after the administration, the mice in the groups were euthanized by cervical spine dislocation [pain category: B], followed by the extraction of tissues. The extracted tissues and the mice from which the tissues were extracted were appropriately disposed of. Since it had been confirmed that the ND6 mutation is maternally inherited to progeny with a probability of 100%, all the mice obtained in this research were analyzed.

### Mitochondria transfer efficiency

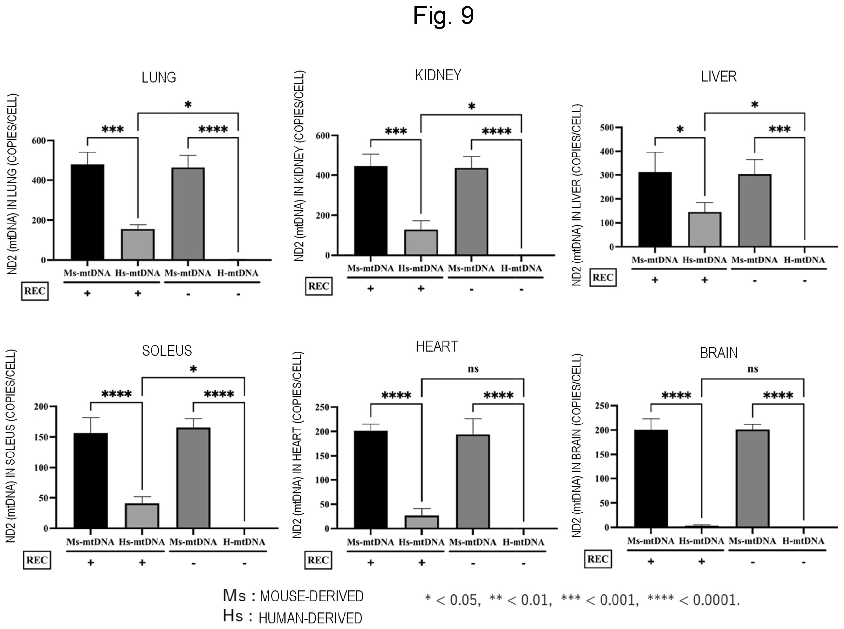

One week after the REC administration (four mice in the treated group and four mice in the control group) and two weeks after the REC administration (four mice in the treated group and four mice in the control group), tissues of target organs (lung/kidney/liver/soleus/heart/brain) were extracted, followed by genome analysis (qPCR; copy number analysis), which measures the copy numbers of the ND2 genes derived from mouse mitochondrial DNA (Ms-mtDNA) and human mitochondrial DNA (H-mtDNA, derived from REC) to inspect the transfer efficiency of human mtDNA (REC-derived mitochondria). Figure 9 shows the results one week after the REC administration.

### Mitochondrial respiratory function

Mitochondria were isolated from the tissues one week after the REC administration (four mice in the treated group and four mice in the control group) and two weeks after the REC administration (four mice in the treated group and four mice in the control group) and measured for mitochondrial respiratory function with a flux analyzer in the same way as in Example 1, to measure the enzymatic activity of the respiratory chain complex.

## Claims

1. A composition for treating and/or preventing mitochondrial disease, comprising a cell population of an LNGFR (CD271)-positive or LNGFR (CD271)/Thy-1 (CD90) double-positive rapidly proliferating mesenchymal stem cell clone or progeny thereof.

2. The composition according to claim 1, wherein the mitochondrial disease is MELAS (mitochondrial myopathy, encephalopathy, lactic acidosis, and stroke-like episodes).

3. The composition according to claim 1 or 2, wherein a coefficient of variation of forward scatter from the cell population or the progeny thereof by flow cytometry is 40% or less.

4. The composition according to claim 1, wherein the cell population is derived from human bone marrow.

5. The composition according to claim 1, wherein the cell population is obtained by a method, comprising:
(a) obtaining a population of LNGFR (CD271)-positive or LNGFR (CD271)/Thy-1 (CD90) double-positive mesenchymal stem cells;
(b) subjecting clones of the mesenchymal stem cells to single-cell culture; and
(c) collecting a clone that proliferates rapidly and subjecting the clone to expansion culture.

6. The composition according to claim 5, wherein the method further comprises: (d) measuring a coefficient of variation of forward scatter by flow cytometry, and (e) collecting a population wherein the coefficient of variation of forward scatter by the flow cytometry is 40% or less.

7. The composition according to claim 1, wherein the composition is intravenously administered to a subject.

8. An agent for improving mitochondrial dysfunction, comprising a cell population of an LNGFR (CD271)-positive or LNGFR (CD271)/Thy-1 (CD90) double-positive rapidly proliferating mesenchymal stem cell clone or progeny thereof.
